# EUROPEAN PATENT SPECIFICATION

(11) **EP 3 544 558 B1**
(45) Date of publication and mention of the grant of the patent: **01.05.2024**
(21) Application number: 17874473.6
(22) Date of filing: 21.11.2017
(51) Int. Cl.: B01D 69/02, B01D 71/80, B01D 69/10, A61F 13/00, B01D 69/12

(54) **WOUND TREATING SYSTEM AND METHODS OF USING AND ASSEMBLING**
WUNDBEHANDLUNGSSYSTEM UND VERFAHREN ZUR VERWENDUNG UND ANORDNUNG
SYSTÈME DE TRAITEMENT DE PLAIE ET PROCÉDÉS D'UTILISATION ET D'ASSEMBLAGE

(30) Priority: 28.11.2016 US 201662426935 P
(43) Date of publication of application: 02.10.2019
(73) Proprietor: Terapore Technologies, Inc., South San Francisco, CA 94080 (US)
(72) Inventor: Ho, John, Sudbury, MA 01776 (US); Dorin, Rachel M., Redwood City, CA 94070 (US)
(74) Representative: Appleyard Lees IP LLP
(86) International application number: PCT/US2017/062681
(87) International publication number: WO 2018/098108

(56) References cited:
- WO-A1-2017/189697
- US-A1- 2006 085 062
- US-A1- 2006 094 598
- US-A1- 2009 208 726
- US-B2- 6 503 958
- US-B2- 8 025 960

## Description

### FIELD OF THE INVENTION

Sterile articles that enable air to contact a wound while contemporaneously regulating moisture in the wound and providing pathogen resistance.

### BACKGROUND OF THE INVENTION

In the health care industry, one of the biggest issues facing healthcare providers is their ability to address chronic or severe wounds, which typically can take months to heal. Currently, the state of the art bandage is a hydrocolloid technology, which is a cellulose-based adhesive that acts as a synthetic scab. The problem with hydrocolloid bandages is that they are made with an occlusive polyurethane backing, which causes maceration (dissolution of tissue due to excessive moisture around the wound site). Healthcare providers therefore need to frequently change bandages to prevent maceration, however frequent dressing changes increases the risk of infection and can disturb the body's natural wound healing process.

Another approach to treating severe wounds is the use of multiple layers of gauze and medical tape. The gauze promotes breathability, but they quickly get saturated with fluids over time and are not resistant to pathogens on the nanoscale. So, healthcare providers must regularly change gauze dressings as well (typically every 8 hours).

US 2009/208726 discloses a device for controlled release of biopharmaceuticals comprising a nanoporous membrane.

There remains a need to provide a tailored, sterile bandage that facilitates air transport to the wound, and at the same time, regulates moisture in the wound to avoid or eliminate maceration (water dissolving tissue around the wound site), for less frequent bandage changes and accelerated healing. There also remains a need to provide broad spectrum bacteria and pathogen resistance without the use of antibacterial coatings that can encourage the growth of antibiotic-resistant strains.

### BRIEF DESCRIPTION OF THE DRAWINGS

FIG. 1 illustrates the multilayer composite bandage of the invention;
FIG. 2 is scanning electron microscope (SEM) image of a nanoporous block copolymer membrane showing highly uniform pore size distribution of the invention.
FIG. 3 is illustrates a further embodiment of the present invention including two distinct membranes with tunable pore size.

### DETAILED DESCRIPTION OF THE INVENTION

The present invention provides a flexible composite bandage, comprising: a) an isoporous, self-assembled nanoporous membrane; b) a flexible and/or elastic support; and c) a biocompatible non-stick agent in at least one of the isoporous membrane and support, as defined in the appended claims. The present invention also provides a kit comprising such bandages and a method of providing a wound treating system, as defined in the appended claims. The tailored articles include external bandages, artificial skin grafts, internal bandages for hernias, and cell scaffolding.

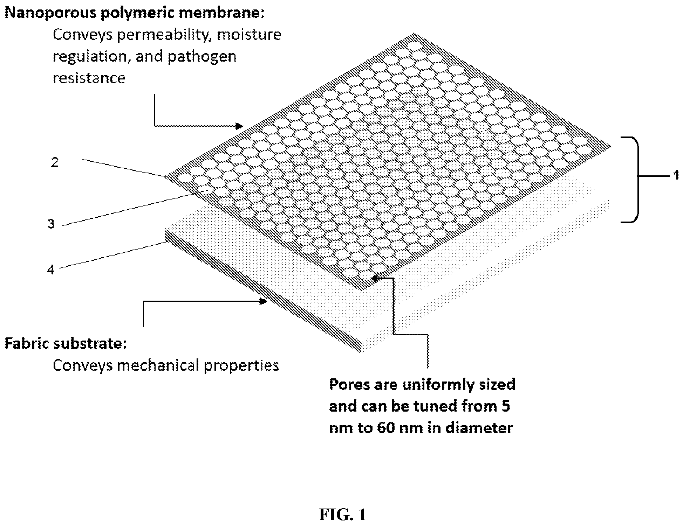

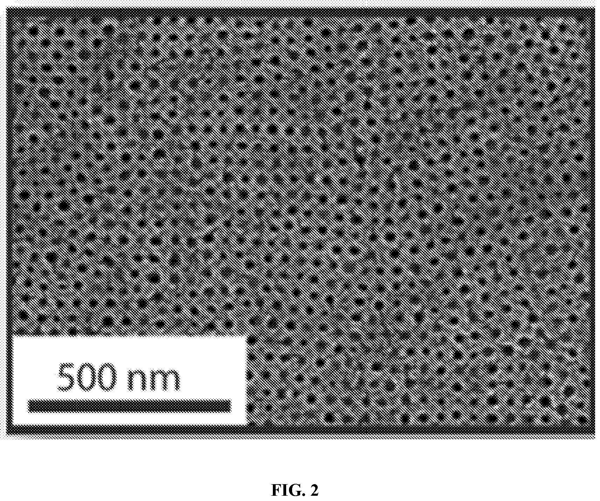

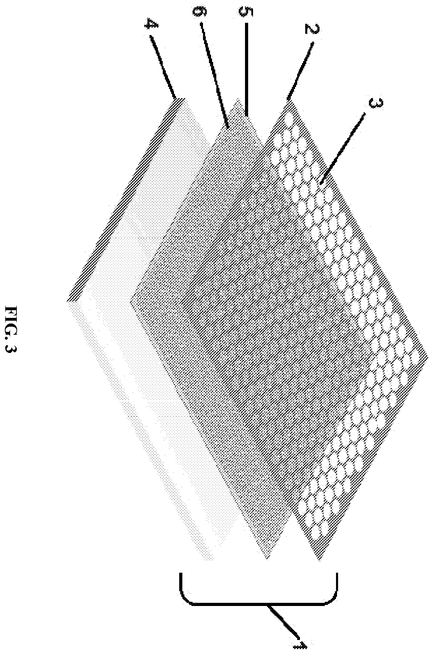

As shown in FIGS. 1 and 3, the article 1 (composite or bandage) includes a membrane 2, tuned pores 3, and support 4. FIG. 3 shows the addition of a second membrane 5, with tuned pores 6 different in size than pores 3. FIG. 2 is an SEM image of the surface of the membrane 2 surface.

More specifically the present inventions provide bandages and kits including isoporous, self-assembled nanoporous membrane having tuned pore sizes ranging from 1 to 60, 100, 200 nm and at least one layer of a flexible, resilient, and/or elastic support layer, which can also be biocompatible. At least one of the support and membrane layers includes an agent that minimizes/reduces sticking to the wound, and can be provided by coating, spraying, or directly incorporating the agent with at least one of the isoporous membrane and support layers. In the context of the invention, isoporous means having a substantially narrow pore diameter distribution.

The membranes of the present invention can be biocompatible for contact with skin/wound or internal use, can be formed from polymeric components that render them biodegradable for temporary use (i.e. synthetic scab), can include antibacterial/microbial agents for sterilizing a wound, can include non-stick agents for release from wound site, can include stiction agents or coating for adhesion to external or internal tissue, can include agents and provide a pore size suitable for enhancing cell growth, and can include drug eluting small molecule(s) and/or biologic(s).

The additional functionality of the membrane of the present invention can include biocompatibility, antibacterial/microbial activity, wound release, drug elution, cell growth enhancement and can be provided by thin film coatings (e.g., dip coating, spray coating, vapor deposition), surface modification (e.g., covalent modification, grafting both to and from, electrostatic attraction (e.g. layer-by-layer, small molecule electrostatic adhesion), varying the membrane polymer chemistry, gas cluster ion beam surface modification, and pore size tunability.

When more than one self-assembled nanoporous membrane are combined, each membrane layer can each have a pore size ranging from 1 to 60 nm, or discrete layers can have a specific pore or different pore size ranges (e.g., 1-30 nm, 10-40 nm, 20-50 nm, 20-40 nm, 20-60 nm). Layers of different size pore ranges/sizes can be combined for additional functionality.

In the present invention, a composite bandage system can have a fabric with multiple layers, each layer providing specific properties. At a minimum, the fabric must contain a tailored nanoporous, polymeric membrane (providing breathability ranging from greater than about 960 g/m² to less than 3200 g/m², regulation of moisture, and pathogen resistance), and a porous backing material that provides mechanical/structural properties, e.g., a support. The bandage system can be provided as a continuous roll, scored or unscored. The bandage fabric support can be longitudinally elastic and tearable in warp or weft direction. Alternatively the bandage system can be provided in discrete sizes of any dimension, e.g., 2 x 2", 2 x 4", 4 x 4", 6 x 8".

The bandage system of the present invention can also be provided as a kit where pathogen specific bandages can be provided as an assortment of pre-assembled bandages, for specific classes of pathogens, specific pathogen species, and specific pharmaceutical treating agents that can also be tailored for a patient or type of wound. The kit includes different pathogen specific bandages, such as, at least one first bandage where isoporous, self-assembled nanoporous membrane having a pore size of from 1 to 60 nm, at least one second bandage with an isoporous, self-assembled nanoporous membrane with a pore size of from 1 to 100 nm, and additional bandages with an isoporous, self-assembled nanoporous membrane having other pore sizes, including at least a pore size of from 1 to 200 nm. The bandages of the kit can provide at least one of biocompatibility, biodegradability, anti-bacterial/microbial, non-stick, cell growth enhancement and drug elution.

The bandage, whether designed for a broad spectrum of pathogens, or tailored to a specific pathogen would be selected and applied to a wound such that either the tuned nanoporous membrane or support layer would face/contact the wound, in which case the materials used for the facing layer should be biocompatible, non-leaching, and able to come in direct contact with the open wound, shown in Figure 1. A bio-compatible non-sticking agent, such as a silicone resin, could be is with either the membrane or support to allow either side of the bandage system to contact a wound.

The nanoporous, polymeric membrane layers should be highly permeable to air, but impervious to pathogens. Pathogens can include bacteria, viruses, protozoa, or algae, generally 0.01 to 0.1 µm in size for most viruses and 0.1 to 20 µm in size for bacteria. Bacteria are spherical (cocci), rod (bacilli) or spiral (spirochetes) in shape, and are also classified as being gram-positive, e.g., Staphylococcus aureus, non-haemolytic streptococci, Beta-haemolytic streptococcus; gram-negative, e.g., Escherichia coli, Pseudomonas aeruginosa, Klebsiella species, Proteus species. Other pathogens includes anerobes, e.g., Bacteroides and Clostridium; and fungi, e.g., Candida albicans and Aspergillus.

Air permeability and pathogen impermeability is achieved through the use of self-assembled block copolymers that form isoporous membranes with pore sizes ranging from 1 to 60 nm, shown in the SEM of Figure 2. The high density of pores gives the membrane higher permeability while the isoporosity acts as a size selection barrier for various pathogens of interest. The pore sizes can also be uniformly tuned to control moisture transmission above the wound site. The membrane is a multi-block copolymer having at least one hydrogen-bonding block and a hydrophobic block. Suitable hydrogen bonding blocks include, but are not limited to, polyvinylpyridines, polyethylene oxides, polyacrylates and polymethacrylates, as well as lower alkyl substituted polyacrylates and polymethacrylates. Suitable hydrophobic blocks can include, but are not limited to polystyrenes, e.g., polystyrene and poly (alkyl substituted styrene), such as, poly (alpha-methyl styrene); polyethylenes, polypropylenes, polyvinyl chlorides, and polytetrafluoroethylenes including expanded PTFE.

The porous backing material simply acts as a mechanical substrate for the nanoporous, polymeric membrane. It should have pore sizes that are much larger than the polymeric membrane so as not to create a bottleneck for permeability, and it should convey mechanical stability and flexibility to the composite bandage. Suitable materials could include a knitted, woven or nonwoven material, such as gauze, cellulose-based fabrics, cotton, rayon, polyesters, polyethylenes, and open structure polyurethane film, all of which are sufficiently breathable to allow air to easily flow to and access the site of a wound.

Typical bandage sizes could match existing standard sizes (e.g. 4 x 4" or 6 x 8") or could be envisioned as a wrap with some self-adhesive material. Alternatives include various material compositions for the various layers, thicknesses of the various layers, as well as variations in the pore size range and degree of isoporosity in the membrane layer. Additional layers can also be added to the stack to provide additional functionality (e.g. biocompatibility, medicinal properties, etc.)

Parameters that provide the necessary protection are the use of a nanoporous, polymeric membrane as a bandage material for wound care. The pore size can range from a few nm and up, which will be important to define control of moisture, transmission rates and rejection of various pathogens.

The solvent 1,4-dioxane, alone or combined with tetrahydrofuran (THF), methanol, ethanol, toluene, chloroform, dimethylformamide, acetone, and dimethylsulfoxide, is used as the solvent in preparing an isoporous graded film of multi-block copolymers, and results in thin selective film layer (i.e., a surface layer) having on the order of more than 10¹⁴ nearly monodisperse mesopores/m² above a graded microporous layer.

Hybridization of the isoporous films via homopolymer or small molecule blending enables tuning of pore size, and can result in pure water flux, solute rejection characteristics, and water vapor transport rates (WVTRs). Tuning pore size is accomplished by the incorporation of small molecules, including but not limited to pentadecyl phenol, dodecyl phenol, 2-4'-(hydroxybenzeneazo) benzoic acid (HABA). 1,8-naphthalene-dimethanol, 3-hydroxy-2-naphthoic acid, and 6-hydroxy-2-naphthoic acid; inorganic and organic acids, including but not limited to hydrofluoric acid, hydrochloric acid, nitric acid, formic acid, acetic acid, propionic acid, lower alkyl di-carboxylic acids; bases, including but not limited to pyridine, ammonia, ammonium hydroxide, sodium hydroxide, potassium hydroxide, amines, polyamines (triethylamine, triethanolamine), amides (acetamide, formamide); including, but not limited to glycerol and other polyols, quinones, hydroquinones, catechols, carbohydrate; and small polymers including but not limited to poly acrylic acid, polyvinylpyridine, polyethylene oxide, naturally-derived polymers (cellulose, chitosan, complex carbohydrates)

In an embodiment, at least one of the membrane or support is coated or impregnated with at least one topical antibiotic. For example, neomycin, polymixin B, mupirocin, bacitracin, erythromycin, or sulfacetamide sodium.

In an embodiment, at least one of the membrane or support is coated or impregnated with at least one clotting agent. For example, thrombin, tannins, metal salts such as zinc and calcium, gelatin, collagen, fibrin, or other blood factors.

In an embodiment, at least one of the membrane or support is coated or impregnated with at least one agent to promote healing. For example, vitamins, proteins, amino acids, enzymes, or drugs. More specifically, some examples include: aloe vera gel or extract, vitamin A, vitamin B1, vitamin B3, glycine, choline salicylate, or collagen.

In an embodiment, at least one of the membrane or support is coated or impregnated with at least one time released drug. For example, compounds for enabling time release or controlled release include: hydroxypropyl methylcellulose, poly(vinyl alcohol), poly(acrylic acid), or waxes.

In an embodiment, at least one of the membrane or support is coated or impregnated with at least one responsively released drug. For example, a drug is released upon exposure to a particular protein or pathogen.

In an embodiment, at least one of the membrane or support is coated or impregnated with at least one antiseptic. For example, benzalkonium chloride, chlorhexidine, alexidine, povidone-iodine, benzethonium chloride, chloroxylenol, alcohols, or triclosan.

In an embodiment, at least one of the membrane or support is coated or impregnated with at least one anesthetic. For example, benzocaine, lidocaine, tetracaine, pramoxine, phenol, menthol, prilocaine, or dyclonine.

In an embodiment, the invention includes an adhesive for affixing the bandage to the body. In some examples, the adhesive is a pressure sensitive adhesive. For example, at least one pressure sensitive adhesive polymer or block copolymers, comprising, for example: poly(acrylates), poly(methacrylates), rubbers, poly(isoprene), poly(butadiene), poly(acrylates), poly(acrylic acid), poly(vinyl acetate), etc.

In an embodiment, the invention is used as a bandage for a burn.

In an embodiment, the invention is used as an oral bandage.

In an embodiment, the invention is used as a bandage for internal body use.

In an embodiment, the bandage is bioabsorbable after a certain period of time. For example, the materials comprise bioabsorbable polymers or copolymers comprising polymers or polymer blocks such as: poly(lactic acid)s, poly(glycolic acid), polyesters, poly(caprolactone), poly(orthoester), poly(hydroxybutyrate valerate), poly(dioxanone), or poly(trimethylene carbonate).

In some embodiments, the bandage has a two-dimensional or three-dimensional geometric arrangement suitable for particular use or body part. For example, a contoured bandage to more appropriately fit a j oint.

In an embodiment, at least one of the membrane or support is coated or impregnated with at least one inactive ingredient for storing, diluting, or delivering a coating or impregnation. For example, pure water, glycerol, petroleum jelly, or lanolin.

In an embodiment, at least one of the membrane or support is coated or impregnated with at least one antioxidant for stability and shelf life. For example, ascorbic acid, tocopherols, carotenoids, carotenes, or butylated hydroxytoluene.

In an embodiment, a portion of the bandage has an inlet, valve, or septum such that a substance can be introduced or extracted to or from the bandage or wound without necessitating removing the bandage. In an example, a drug can be injected from a syringe either with or without a needle without removing the bandage. In an example, a sample of bodily fluid can be extracted with a syringe, with or without a needle, from the bandage or wound without removing the bandage.

In an embodiment, the bandage is directly inserted into a wound as a packing.

In an embodiment, the bandage incorporates an indicator. Said indicator, for example, may indicate: a bandage needs changing, lack of moisture, excess moisture, presence of a pathogen, expenditure of drug or topical agent, wound coagulation, etc.

In an embodiment, the bandage is packaged with a hydrating agent or humectant to retain moisture on the membrane. For example, pure water, glycerol, or aloe vera gel.

In an embodiment, the bandage is packaged dry.

## Claims

1. A flexible composite bandage, comprising:
a) an isoporous, self-assembled nanoporous membrane;
b) a flexible and/or elastic support; and
c) a biocompatible non-stick agent in at least one of the isoporous membrane and support.

2. The bandage of claim 1, wherein the isoporous, self-assembled nanoporous membrane has pore sizes ranging from 1 to 200 nm; and wherein at least one of the membrane and the support are biocompatible.

3. The bandage of claim 1, wherein the isoporous, self-assembled nanoporous membrane has pore sizes ranging from 1 to 60 nm; and wherein at least one of the membrane and the support are biocompatible.

4. A method of providing a wound treating system, the method comprising associating an isoporous self- assembled nanoporous membrane having pores with sizes ranging from 1 to 200 nm to a flexible and/or elastic support to form a unitary bandage, wherein
a biocompatible non-stick agent is associated with at least one of the isoporous self-assembled nanoporous membrane and the support; and
at least one of the membrane and support are biocompatible.

5. The bandage of claim 1, wherein the support comprises at least one layer of an air permeable, self-supporting, biocompatible material.

6. The bandage of claim 1, wherein the isoporous membrane is arranged on an outmost layer of the bandage.

7. The bandage of claim 5, wherein the support includes a plurality of layers and the isoporous membrane is arranged between two of the plurality of layers.

8. The bandage according to claim 1, wherein a biocompatible non-stick agent is coated on a surface of the isoporous membrane and support such that the non-stick agent contacts a wound when the bandage is in use.

9. The bandage according to claim 1, wherein the bandage is pathogen specific.

10. The bandage according to claim 1, wherein at least one of the membrane and support include a pharmaceutical agent or a pharmaceutical drug.

11. A kit, the kit comprising a bandage according to any one of claims 1 to 3 and 5 to 10.

12. A kit comprising different pathogen specific bandages according to any one of claims 1 to 3 and 5 to 10, the different pathogen specific bandages comprising:
at least one first bandage comprising an isoporous, self-assembled nanoporous membrane having a pore size of from 1 to 60 nm,
at least one second bandage comprising an isoporous, self-assembled nanoporous membrane having a pore size of from 1 to 100 nm, and
at least one third bandage comprising an isoporous, self-assembled nanoporous membrane having other pore sizes, including at least a pore size of from 1 to 200 nm.

13. The kit of claim 12, where at least one of the bandages provides at least one of biocompatibility, biodegradability, anti-bacterial/microbial, non-stick, cell growth enhancement and drug elution.

## Patentansprüche

1. Flexibler Verbundstoffverband, umfassend:
a) eine isoporöse selbstorganisierte nanoporöse Membran;
b) einen flexiblen und/oder elastischen Träger; und
c) ein biokompatibles Antihaftmittel in mindestens einem von der isoporösen Membran und dem Träger.

2. Verband nach Anspruch 1, wobei die isoporöse selbstorganisierte nanoporöse Membran Porengrößen im Bereich von 1 bis 200 nm aufweist; und wobei mindestens eines von der Membran und dem Träger biokompatibel ist.

3. Verband nach Anspruch 1, wobei die isoporöse selbstaufbauende nanoporöse Membran Porengrößen im Bereich von 1 bis 60 nm aufweist; und wobei mindestens eines von der Membran und dem Träger biokompatibel ist.

4. Verfahren zum Bereitstellen eines Wundbehandlungssystems, wobei das Verfahren das Vereinigen einer isoporösen selbstorganisierten nanoporösen Membran, die Poren mit Größen im Bereich von 1 bis 200 nm aufweist, mit einem flexiblen und/oder elastischen Träger unter Bildung eines einheitlichen Verbands umfasst, wobei
ein biokompatibles Antihaftmittel mit mindestens einem von der isoporösen selbstorganisierten nanoporösen Membran und dem Träger vereinigt wird; und
mindestens eines von der Membran und dem Träger biokompatibel ist.

5. Verband nach Anspruch 1, wobei der Träger mindestens eine Schicht aus einem luftdurchlässigen selbsttragenden biokompatiblen Material umfasst.

6. Verband nach Anspruch 1, wobei die isoporöse Membran auf einer äußersten Schicht des Verbands angeordnet ist.

7. Verband nach Anspruch 5, wobei der Träger eine Vielzahl von Schichten umfasst und die isoporöse Membran zwischen zwei der Mehrzahl von Schichten angeordnet ist.

8. Verband nach Anspruch 1, wobei ein biokompatibles Antihaftmittel auf eine Oberfläche der isoporösen Membran und des Trägers aufgetragen ist, sodass das Antihaftmittel bei Verwendung des Verbands in Kontakt mit einer Wunde kommt.

9. Verband nach Anspruch 1, wobei der Verband pathogenspezifisch ist.

10. Verband nach Anspruch 1, wobei mindestens eines von der Membran und dem Träger ein pharmazeutisches Mittel oder ein pharmazeutisches Arzneimittel umfasst.

11. Kit, wobei das Kit einen Verband nach einem der Ansprüche 1 bis 3 und 5 bis 10 umfasst.

12. Kit, umfassend unterschiedliche pathogenspezifische Verbände nach einem der Ansprüche 1 bis 3 und 5 bis 10, wobei die unterschiedlichen pathogenspezifischen Verbände Folgendes umfassen:
mindestens einen ersten Verband, der eine isoporöse selbstorganisierte nanoporöse Membran mit einer Porengröße von 1 bis 60 nm umfasst,
mindestens einen zweiten Verband, der eine isoporöse selbstorganisierte nanoporöse Membran mit einer Porengröße von 1 bis 100 nm umfasst, und
mindestens einen dritten Verband, der eine isoporöse selbstorganisierte nanoporöse Membran mit einer anderen Porengröße, einschließlich zumindest einer Porengröße von 1 bis 200 nm, umfasst.

13. Kit nach Anspruch 12, wobei mindestens einer der Verbände mindestens eines von Biokompatibilität, Bioabbaubarkeit, antibakteriell/mikrobiell, Antihaft-, Zellwachstumsverstärkung und Arzneimittelelution bereitstellt.

## Revendications

1. Bandage composite flexible, comprenant :
a) une membrane nanoporeuse auto-assemblée isoporeuse ;
b) un support flexible et/ou élastique ; et
c) un agent antiadhésif biocompatible dans au moins l'un de la membrane isoporeuse et du support.

2. Bandage selon la revendication 1, la membrane nanoporeuse auto-assemblée isoporeuse ayant des tailles de pore dans la plage de 1 à 200 nm ; et au moins l'un de la membrane et du support étant biocompatibles.

3. Bandage selon la revendication 1, la membrane nanoporeuse auto-assemblée isoporeuse ayant des tailles de pore dans la plage de 1 à 60 nm ; et au moins l'un de la membrane et du support étant biocompatibles.

4. Procédé pour fournir un système de traitement des plaies, le procédé comprenant l'association d'une membrane nanoporeuse auto-assemblée isoporeuse ayant des tailles de pore dans la plage de 1 à 200 nm à un support flexible et/ou élastique pour former un bandage unitaire, un agent antiadhésif biocompatible étant associé à au moins l'un de la membrane nanoporeuse auto-assemblée isoporeuse et du support ; et
au moins l'un de la membrane et du support étant biocompatibles.

5. Bandage selon la revendication 1, le support comprenant au moins une couche d'un matériau perméable à l'air, autoportant et biocompatible.

6. Bandage selon la revendication 1, la membrane isoporeuse étant disposée sur une couche la plus extérieure du bandage.

7. Bandage selon la revendication 5, le support comprenant une pluralité de couches et la membrane isoporeuse étant disposée entre deux de la pluralité de couches.

8. Bandage selon la revendication 1, un agent antiadhésif biocompatible étant enduit sur une surface de la membrane isoporeuse et du support de telle sorte que l'agent antiadhésif entre en contact avec une plaie lorsque le bandage est utilisé.

9. Bandage selon la revendication 1, le bandage étant spécifique à un agent pathogène.

10. Bandage selon la revendication 1, au moins l'un de la membrane et du support comprenant un agent pharmaceutique ou un médicament pharmaceutique.

11. Kit, le kit comprenant un bandage selon l'une quelconque des revendications 1 à 3 et 5 à 10.

12. Kit comprenant différents bandages spécifiques aux agents pathogènes selon l'une quelconque des revendications 1 à 3 et 5 à 10, les différents bandages spécifiques aux agents pathogènes comprenant :
au moins un premier bandage comprenant une membrane nanoporeuse auto-assemblée isoporeuse dont la taille des pores est comprise entre 1 et 60 nm,
au moins un deuxième bandage comprenant une membrane nanoporeuse auto-assemblée isoporeuse dont la taille des pores est comprise entre 1 et 100 nm, et
au moins un troisième bandage comprenant une membrane nanoporeuse auto-assemblée isoporeuse ayant d'autres tailles de pores, comprenant au moins une taille de pore comprise entre 1 et 200 nm.

13. Kit selon la revendication 12, au moins un des bandages fournissant au moins une des caractéristiques suivantes : biocompatibilité, biodégradabilité, anti-bactérien/microbien, anti-adhérence, amélioration de la croissance cellulaire et élution de médicament.
